# EUROPEAN PATENT APPLICATION

(11) **EP 4 488 372 A2**
(43) Date of publication of application: **08.01.2025**
(21) Application number: 24211666.3
(22) Date of filing: 26.09.2019
(51) Int. Cl.: C12N 15/57

(54) **METHOD FOR ENHANCING SENSITIVITY OF ENDOTOXIN MEASURING AGENT**

(30) Priority: 03.10.2018 JP 2018188587
(62) Divisional of application: 19869215.4
(71) Applicant: Seikagaku Corporation, Tokyo 100-0005 (JP)
(72) Inventor: OGURA, Norihiko, Tokyo 100-0005 (JP); KOBAYASHI, Yuki, Tokyo 100-0005 (JP); MIZUMURA, Hikaru, Tokyo 100-0005 (JP); ODA, Toshio, Tokyo 100-0005 (JP)
(74) Representative: Müller-Boré & Partner Patentanwälte PartG mbB

(57) **Abstract**

The present invention relates to a method for enhancing a sensitivity of an endotoxin measuring reagent to the endotoxin of *Helicobacter pylori,* the endotoxin measuring reagent comprising a recombinant protein of horseshoe crab factor C, a recombinant protein of horseshoe crab factor B, and a recombinant protein of horseshoe crab pro-clotting enzyme, the method comprising increasing a content of the recombinant protein of factor C to 162 ng/mL or more at the time of endotoxin measurement.

## Description

### Technical Field

The present invention relates to a method for enhancing the sensitivity of an endotoxin measuring reagent to the endotoxin of Helicobacter pylori.

### Background Art

It is known that an extract of amoebocyte present in blood of a horseshoe crab (amoebocyte lysate) coagulates when it contacts with an endotoxin. By taking advantage of this property, the amoebocyte lysate is widely used, as a reagent for detecting an endotoxin at high sensitivity, for quality management of a pharmaceutical product or the like.

The reagent is referred to as a "lysate reagent". Furthermore, as the property of causing coagulation by an endotoxin is initially found in Limulus polyphemus, there are also cases in which the reagent is referred to as a "Limulus reagent" or a "Limulus amoebocyte lysate (LAL) reagent".

For the protection of a horseshoe crab, there is an idea of producing the reagent by artificially produced proteins in an amoebocyte lysate based on recombination techniques (Patent Literatures 1 and 2). In fact, by using recombinant proteins, products like PyroGene (registered trademark) (LONZA JAPAN), EndoZyme (registered trademark) II (bioMérieux Japan Ltd.), and PyroSmart (registered trademark) (SEIKAGAKU CORPORATION) have been launched onto the market.

From the results of Ministry of Health, Labor, and Welfare Grants (Non Patent Literature 1) by Tanamoto et al. and reports by Loverock et al. (Non Patent Literature 2), Grallert et al. (Non Patent Literature 3), and Bolden et al. (Non Patent Literature 4), it was considered that the performance of the "recombinant reagent", which is an endotoxin measuring reagent using recombinant proteins, is equivalent to the lysate reagent.

### Citation List

### Patent Literature

Patent Literature 1: WO 2012/118226 A
Patent Literature 2: WO 2014/092079 A

### Non Patent Literature

Non Patent Literature 1: Document Number 201427027B of the results of Ministry of Health, Labor, and Welfare Grants "Research on Introduction of Advanced Test Method for Securing Microbiological Quality of Pharmaceutical Product"
Non Patent Literature 2: Loverock, B et al., "A Recombinant Factor C Procedure for the Detection of Gram-Negative Bacteria Endotoxina", Pharmacopeial Forum 36, 321-29, (2010)
Non Patent Literature 3: Grallert, H et al., "EndoLISA: a novel and reliable method for endotoxin detection", Nature Methods, October (2011)
Non Patent Literature 4: Bolden, J. et al., "Application of Recombinant Factor C Reagent for the Detection of Bacteria Endotoxins in Pharmaceutical Products", PDA J Pharm Sci Technol., 2017, Sep-Oct, 71(5), 405-412 (2017)

### Summary of Invention

However, as a result of measuring the relative potency of the endotoxin of Helicobacter pylori in comparison with that of Reference Standard Endotoxin by using a current recombinant reagent, the inventors of the present invention found that the relative potency is significantly lower than that measured by using a lysate reagent. Accordingly, an object of the present invention is to provide a method for enhancing the sensitivity of an endotoxin measuring reagent using a recombinant protein to the endotoxin of Helicobacter pylori.

As a result of carrying out intensive studies to solve the above problems, the inventors of the present invention found that, by increasing a content of a recombinant protein of factor C in an endotoxin measuring reagent employing a recombinant protein at the time of endotoxin measurement (namely, co-existing with the endotoxin of Helicobacter pylori), the sensitivity to the endotoxin of Helicobacter pylori can be enhanced. Based on these findings, the inventors of the present invention completed the present invention.

Namely, the present invention relates to the followings.
[1] A method for enhancing a sensitivity of an endotoxin measuring reagent to the endotoxin of Helicobacter pylori (endotoxin derived from Helicobacter pylori),
   the endotoxin measuring reagent containing a recombinant protein of horseshoe crab factor C (factor C derived from a horseshoe crab),
   the method including increasing a content of the recombinant protein of factor C at the time of endotoxin measurement.
[2] The method described in [1], in which the endotoxin measuring reagent contains a recombinant protein of horseshoe crab factor B (factor B derived from a horseshoe crab).
[3] The method described in [1] or [2], in which the endotoxin measuring reagent contains a recombinant protein of horseshoe crab pro-clotting enzyme (pro-clotting enzyme derived from a horseshoe crab).
[4] The method described in any one of [1] to [3], in which the endotoxin measuring reagent contains a compound for detection.
[5] An endotoxin measuring method in which sensitivity of an endotoxin measuring reagent to the endotoxin of Helicobacter pylori is enhanced, the method including:
   preparing an endotoxin measurement sample containing the endotoxin measuring reagent and an analyte,
   in which the endotoxin measuring reagent contains a recombinant protein of horseshoe crab factor C, and
   the sensitivity is enhanced by increasing a concentration of the recombinant protein of factor C in the endotoxin measurement sample.
[6] The method described in [5], in which the endotoxin measuring reagent contains a recombinant protein of horseshoe crab factor B.
[7] The method described in [5] or [6], in which the endotoxin measuring reagent contains a recombinant protein of horseshoe crab pro-clotting enzyme.
[8] The method described in any one of [5] to [7], in which the endotoxin measuring reagent contains a compound for detection.
[9] An endotoxin measuring reagent having enhanced sensitivity to the endotoxin of Helicobacter pylori.
[10] A method for producing an endotoxin measuring reagent having enhanced sensitivity to the endotoxin of Helicobacter pylori,
   the endotoxin measuring reagent containing a recombinant protein of horseshoe crab factor C,
   the method including increasing a content of the recombinant protein of factor C in the endotoxin measuring reagent.
[11] An endotoxin measuring reagent containing a horseshoe crab factor C, in which the horseshoe crab factor C is a recombinant protein, and relative potency of the endotoxin of Helicobacter pylori is 0.1 times or more relative potency measured by using a lysate reagent.
[12] The endotoxin measuring reagent described in [11], in which the endotoxin measuring reagent contains a recombinant protein of horseshoe crab factor B.
[13] The endotoxin measuring reagent described in [11] or [12], in which the endotoxin measuring reagent contains a recombinant protein of horseshoe crab pro-clotting enzyme.
[14] The endotoxin measuring reagent described in any one of [11] to [13], in which the endotoxin measuring reagent contains a compound for detection.
[15] An endotoxin measuring reagent containing a horseshoe crab factor C, in which the horseshoe crab factor C is a recombinant protein, and relative potency of the endotoxin of Helicobacter pylori is 200 (EU/ug) or more.
[16] The endotoxin measuring reagent described in [15], in which the endotoxin measuring reagent contains a recombinant protein of horseshoe crab factor B.
[17] The endotoxin measuring reagent described in [15] or [16], in which the endotoxin measuring reagent contains a recombinant protein of horseshoe crab pro-clotting enzyme.
[18] The endotoxin measuring reagent described in any one of [15] to [17], in which the endotoxin measuring reagent contains a compound for detection. One aspect of the compound for detection is a compound for detection represented by general formula Y-X-Z, in which in the general formula, Y is a hydrogen atom or a protecting group, X is a peptide containing the amino acid sequence, which is a substrate of the recombinant protein of pro-clotting enzyme, and Z is a labeling material which becomes optically detectable when being released from X. Another aspect of the compound for detection is a compound for detection represented by general formula Y-X-Z, in which in the general formula, Y is a hydrogen atom or a protecting group, X is a peptide containing the amino acid sequence, which is a substrate of the recombinant protein of factor B, and Z becomes a labeling material which is optically detectable when being released from X. Still another aspect of the compound for detection is a compound for detection represented by general formula Y-X-Z, in which in the general formula, Y is a hydrogen atom or a protecting group, X is a peptide containing the amino acid sequence, which is a substrate of the factor C, and Z is a labeling material which becomes optically detectable when being released from X.
[19] A method for measuring endotoxin in an analyte by using the endotoxin measuring reagent described in any one of [11] to [18].

### Brief Description of Drawings

FIG. 1 is a diagram showing the relationship between the concentration of a recombinant factor C in a reaction solution and the relative potency of endotoxin of Helicobacter pylori CA2.
FIG. 2 is a diagram showing the relationship between the concentration of a recombinant factor B in a reaction solution and the relative potency of the endotoxin of Helicobacter pylori CA2.
FIG. 3 is a diagram showing the relationship between the concentration of a recombinant pro-clotting enzyme in a reaction solution and the relative potency of endotoxin of Helicobacter pylori CA2.

### Description of Embodiments

Abbreviations used in the present specification and meanings of the abbreviations are described.
Factor C (FC)
Recombinant factor C (rFC)
Factor B (FB)
Recombinant factor B (rFB)
Pro-clotting enzyme (coagulation enzyme precursor: PCE)
Recombinant pro-clotting enzyme (rPCE)
Clotting enzyme (coagulation enzyme)

According to the present invention, a method for enhancing the sensitivity of an endotoxin measuring reagent employing a recombinant protein to the endotoxin of Helicobacter pylori can be provided.

In the present specification, the factor C, the factor B, and the pro-clotting enzyme may be also referred to as, either individually or together, a "Limulus factor".

The present invention is described hereinbelow.

### <Method for enhancing sensitivity of endotoxin measuring reagent>

One aspect of the present invention relates to a method for enhancing the sensitivity of an endotoxin measuring reagent to the endotoxin of Helicobacter pylori, in which the endotoxin measuring reagent contains a recombinant protein of factor C of horseshoe crab and the method includes increasing a content of the recombinant protein of factor C at the time of endotoxin measurement (hereinbelow, the method may be also referred to as an "enhancing method of the present invention"). According to an embodiment of the present invention, the content of the recombinant protein of factor C at the time of endotoxin measurement is increased to an amount that is sufficient for enhancing the sensitivity.

Another aspect of the present invention relates to a method for improving the reactivity of an endotoxin measuring reagent to the endotoxin of Helicobacter pylori, in which the endotoxin measuring reagent contains a recombinant protein of horseshoe crab factor C, and the method includes increasing a content of the recombinant protein of factor C at the time of endotoxin measurement. Further aspect of the present invention relates to a method for imparting the reactivity of a recombinant protein of factor C of horseshoe crab to the endotoxin of Helicobacter pylori, the method including increasing an amount of the recombinant protein of factor C to co-exist with the endotoxin. In an embodiment of the present invention, the reactivity is expressed as relative potency of 200 EU/µg or higher (for example, 250 EU/µg or higher or 300 EU/µg or higher).

As described in the above, the inventors of the present invention recognized a problem that the sensitivity of a current endotoxin measuring reagent employing a recombinant protein to the endotoxin of Helicobacter pylori is significantly lower than that of a lysate reagent.

According to the enhancing method of the present invention, for measuring an endotoxin in which a recombinant reagent as an endotoxin measuring reagent employing a recombinant protein is used, by increasing the content of the recombinant protein of factor C at the time of endotoxin measurement, it is made possible to enhance the sensitivity of an endotoxin measuring reagent to the endotoxin of Helicobacter pylori. Namely, the relative potency of the endotoxin of Helicobacter pylori to Reference Standard Endotoxin can be enhanced. More specifically, by increasing the concentration of a recombinant protein of factor C in an endotoxin measurement sample which contains an endotoxin measuring reagent and an analyte, the sensitivity of an endotoxin measuring reagent to the endotoxin of Helicobacter pylori can be enhanced. Herein, as described in Examples described below, the inventors of the present invention recognized that, even when the concentration of a Limulus factor other than the recombinant protein of factor C is increased in an endotoxin measurement sample, the relative potency of the endotoxin of Helicobacter pylori with reference to that of Reference Standard Endotoxin cannot be increased or a higher background is caused so that the sensitivity of an endotoxin measuring reagent to the endotoxin of Helicobacter pylori cannot be enhanced.

According to the enhancing method of the present invention, the content of the recombinant protein of factor C that is sufficient for enhancing the sensitivity of an endotoxin measuring reagent at the time of endotoxin measurement can be varied depending on desired relative potency, measurement system, hosts of the recombinant protein of factor C, or the like, and, although not limited, the content of the recombinant protein of factor C can be increased such that the relative potency of the endotoxin of Helicobacter pylori relative to that of Reference Standard Endotoxin, which is measured by using an endotoxin measuring reagent employing a recombinant protein, is 0.1 times or more, 0.2 times or more, 0.3 times or more, 0.4 times or more, 0.5 times or more, 0.6 times or more, 0.7 times or more, 0.8 times or more, 0.9 times or more, or 1.0 times or more the relative potency measured by using a lysate reagent.

Herein, as for the lysate reagent, a hemocyte extract prepared from lymph fluid of horseshoe crab blood by a common method (see, J. Biochem., 80, 1011-1021 (1976), for example) can be mentioned. Furthermore, it is also possible to use the extract added with, if necessary, a divalent metal salt effective for factor C activation, a substrate of a clotting enzyme (for example, synthetic substrate described below), a pH adjusting reagent, and the like. Furthermore, as for the lysate reagent, commercially available products can be also used. The lysate reagent is not limited, and examples thereof include Endospecy (registered trademark) ES-50M (SEIKAGAKU CORPORATION).

Furthermore, measurement of the relative potency of the endotoxin of Helicobacter pylori by using an endotoxin measuring reagent applying a recombinant protein can be achieved by referring Reference Standard Endotoxin, and, for example, the measurement can be carried out according to the method described in Examples using a compound for detection. The measurement method may be either end-point measurement or kinetics measurement.

Specifically, as for measurement of the relative potency, for example, (i) absorbance of an endotoxin measurement sample is measured and an absorbance change ratio per unit time (mAbs/min) is calculated. (ii) Subsequently, from a calibration curve which has been established by using Reference Standard Endotoxin, the endotoxin activity (EU/mL) of an endotoxin measurement sample is calculated, and (iii) by dividing this value by the concentration (µg/mL) of endotoxin contained in an endotoxin measurement sample, the relative potency (EU/µg) of the endotoxin is calculated. The method using a change in absorbance over time is particularly suitable for measurement of the relative potency of endotoxin in a three factor system (a reaction system including a factor C, a factor B, and a pro-clotting enzyme). In an embodiment, the measurement of the relative potency of the endotoxin of Helicobacter pylori is carried out in a three factor system by the method using a change in absorbance over time.

The measurement of the relative potency may be carried out by a method based on fluorescence intensity measurement. More specifically, (i) a change in relative fluorescence intensity of an endotoxin measurement sample during a cascade reaction is measured by using an endotoxin measuring reagent, (ii) an endotoxin activity (EU/mL) of the endotoxin measurement sample is calculated from a calibration curve which has been established by using Reference Standard Endotoxin, and (iii) this value is divided by the concentration (µg/mL) of endotoxin contained in the endotoxin measurement sample to calculate the relative potency (EU/µg) of endotoxin. A method using a change in fluorescence intensity over time is particularly suitable for measurement of the relative potency in a one factor system (a reaction system including a factor C and not including a factor B or a pro-clotting enzyme) and measurement of the relative potency in a two factor system (a reaction system including a factor C and a factor B and not including a pro-clotting enzyme). In an embodiment, the measurement of the relative potency of the endotoxin of Helicobacter pylori is carried out in a one factor system or a two factor system by the method using a change in fluorescence intensity over time.

Herein, the amount of the endotoxin of Helicobacter pylori used in measurement of the relative potency is not particularly limited as long as it is an amount with which the endotoxin activity (EU/mL) of an endotoxin measurement sample (namely, a sample containing the endotoxin of Helicobacter pylori) can be calculated from a calibration curve which has been established by using Reference Standard Endotoxin, and the amount thereof can be suitably set depending on various conditions such as the type of a method of measuring relative potency. The amount of the endotoxin of Helicobacter pylori used in measurement of the relative potency may be, in terms of concentration in a measurement system, for example, 0.25 to 25 ng/mL, and may be specifically 0.25 ng/mL, 5 ng/mL, or 25 ng/mL. The amount of the endotoxin of Helicobacter pylori used in measurement of the relative potency may be, in terms of concentration in a measurement system, 0.25 to 25 ng/mL, and may be specifically 0.25 ng/mL, 5 ng/mL, or 25 ng/mL, particularly in the case of performing measurement of relative potency in a one factor system. In addition, the amount of the endotoxin of Helicobacter pylori used in measurement of the relative potency may be, in terms of concentration in a measurement system, 0.25 ng/mL particularly in the case of performing measurement of relative potency in a two factor system or a three factor system.

Herein, examples of Reference Standard of Endotoxin include a Reference Standard Endotoxin Japanese Pharmacopoeia, Reference Standard Endotoxin of US Pharmacopoeia, and Endotoxin Standard of European Pharmacopoeia.

In a case in which a commercially available recombinant reagent A that is used in Examples described below is used, for example, the relative potency of the endotoxin of Helicobacter pylori is 147.3 EU/µg (Table 1). On the other hand, in a case in which a commercially available lysate reagent A or lysate reagent B is used, the relative potency of endotoxin of Helicobacter pylori is about 2,000 EU/µg. In a case in which an endotoxin is measured based on a three factor system using a recombinant protein of factor C which has been expressed in mammalian cells, if the content of the recombinant protein of factor C at the time of endotoxin measurement (namely, in the measurement sample at the time of endotoxin measurement) is set at 162 ng/mL or more, 0.25 ng/ml of endotoxin of Helicobacter pylori becomes possible to achieve the relative potency of 200 EU/µg or higher, which is 0.1 times or more than the relative potency measured by using a lysate reagent (Table 2). In this case, by the increased content of the recombinant protein of factor C in the measurement sample to 162 ng/mL or more at the time of endotoxin measurement, the sensitivity of an endotoxin measuring reagent to the endotoxin of Helicobacter pylori can be enhanced. Furthermore, in Table 1, the content of the recombinant protein of factor C in a commercially available recombinant reagent A at the time of endotoxin measurement is less than 162 ng/mL.

The content of the recombinant protein of factor C in the measurement sample at the time of endotoxin measurement can be varied depending on desired relative potency, measurement system, hosts of the recombinant protein of factor C, or the like. In a case in which the measurement system is based on a three factor system, although it is not limited, the content of the recombinant protein of factor C in the measurement sample at the time of endotoxin measurement can be set at 140 ng/mL or more, 162 ng/mL or more, 180 ng/mL or more, 200 ng/mL or more, 250 ng/mL or more, 300 ng/mL or more, 400 ng/mL or more, 500 ng/mL or more, 600 ng/mL or more, or 700 ng/mL or more. In a case in which the measurement system is in a two factor system, although it is not limited, the content of the recombinant protein of factor C in the measurement sample at the time of endotoxin measurement can be set at 235 ng/mL or more, 250 ng/mL or more, 300 ng/mL or more, or 350 ng/mL or more. In a case in which the measurement system is a one factor system, although it is not limited, the content of the recombinant protein of factor C in the measurement sample at the time of endotoxin measurement can be set at 10 ng/mL or more, 20 ng/mL or more, 30 ng/mL or more, 40 ng/mL or more, 50 ng/mL or more, 60 ng/mL or more, 70 ng/mL or more, 100 ng/mL or more, 500 ng/mL or more, or 800 ng/mL or more.

The upper limit of the content of the recombinant protein of factor C in the measurement sample at the time of endotoxin measurement is not particularly limited, and is, for example, 10 mg/mL or less and is preferably less than 10 ug/mL (for example, 5 ug/mL or less or 2 ug/mL or less) from the viewpoint of production efficiency.

Furthermore, in the present specification, the content of the recombinant protein of factor C is a value calculated by ELISA method described in Examples.

The method for measuring endotoxin using a Limulus reagent is a method utilizing a progress of cascade reaction in which a serine protease precursor (for example, factor C, factor B, and a pro-clotting enzyme) contained in amoebocyte lysate is gradually activated in contact with endotoxin.

The endotoxin measuring method to which the enhancing method of the present invention is applied can be also a method in which the measurement is carried out by utilizing the protease activity of an active form factor C which is self-catalytically converted from a factor C to the active form (active form factor C) in contact with endotoxin (one factor system). Further, the endotoxin measuring method to be applied in the present invention may be a method of carrying out the measurement by utilizing the reaction of the conversion into an active form factor B as the factor B is cleaved off by the protease activity of the active form factor C which is a factor C self-catalytically converted to the active form in contact with endotoxin (two factor system). Furthermore, the endotoxin measuring method to be applied in the present invention may be a method of carrying out the measurement by utilizing, in addition to those series of reaction, a series of reaction which includes conversion into a clotting enzyme as a pro-clotting enzyme is cleaved off by the protease activity of the active form factor B (three factor system). The "cascade reaction" in the present specification includes the reactions of the one factor system, the two factor system, and the three factor system described above.

The endotoxin measuring method to which the enhancing method of the present invention is applied can be applied without being particularly limited, as long as it is an endotoxin measuring method which utilizes a recombinant reagent as an endotoxin measuring reagent using a recombinant protein of factor C. The recombinant reagent may be either a commercially available recombinant reagent or a reagent composed of a Limulus factor which has been produced by a well-known method.

With regard to the recombinant reagent, the factor C is a recombinant protein of Limulus factor prepared by genetic engineering techniques.

With regard to the above recombinant reagent, the factor B and a pro-clotting enzyme may be, each independently, either a natural Limulus factor obtained from a horseshoe crab, a recombinant protein of a Limulus factor which has been prepared by genetic engineering techniques, or a mixture containing, at an arbitrary ratio, a natural Limulus factor and a recombinant protein of Limulus factor. Preferably, the factor B and the pro-clotting enzyme are recombinant proteins of Limulus factor.

The natural Limulus factor may be those resulting from suitable purification and fractionation of a lysate which is obtained by a common method from hemocytes of a horseshoe crab as a raw material. Fractionation of Limulus factor can be carried out in view of the method described in the literature (Nakamura, T., Horiuchi, T., Morita, T., Iwanaga, S. (1986) J. Biochem. 99, 847-57), for example.

The recombinant protein of Limulus factor can be obtained by producing the Limulus factor in a cell to which a nucleic acid encoding the polypeptide of Limulus factor is introduced. The base sequence of the nucleic acid encoding Limulus factor can be obtained from a known database like NCBI (www.ncbi.nlm.nih.gov). Furthermore, the base sequence of the nucleic acid encoding Limulus factor can be also a variant of DNA of which codon combination is optimized for the expression in host cells.

The type of the horseshoe crab to be an origin of each Limulus factor is not particularly limited. As for the horseshoe crab, those belonging to genus Tachypleus, genus Limulus, or genus Carcinoscorpius can be exemplified. More specifically, four types, that is, Tachypleus tridentatus, Limulus polyphemus, Carcinoscorpius rotundicauda, and Tachypleus gigas, are known. These horseshoe crabs can be exemplified as a horseshoe crab to be an origin of Limulus factor. In addition, among those horseshoe crabs, Tachypleus tridentatus, Carcinoscorpius rotundicauda, or Limulus polyphemus is preferred, and Tachypleus tridentatus.is more preferred.

As for the polypeptide of Limulus factor, the polypeptides of (1) to (8) described in <Method for measuring endotoxin> described below are specifically exemplified. As for the nucleic acid encoding Limulus factor, base sequences encoding polypeptide of [1] to [7] described in <Method for measuring endotoxin> described below are specifically exemplified.

Production of Limulus factor using cells can be carried out by a well-known technique, and specific examples thereof include a method using mammalian cells or non-mammalian cells described in <Example 2> or the like described below (for example, insect cells, yeasts, parasitic protozoan such as Leishmania, or the like). Furthermore, production of Limulus factor using cells can be also carried out in view of the method described in the literature, for example (WO 2018/074498 A).

With regard to the enhancing method of the present invention, the method can be carried out under well-known conditions for measuring endotoxin except that the content of the recombinant protein of factor C at the time of endotoxin measurement is increased, for example, to an amount that is sufficient for the enhancement of sensitivity. The conditions for measurement are not limited, but, as for the pH of a reaction solution, the conditions with a pH of 5 to 10 and preferably 7 to 8.5 can be employed, for example. As for the reaction temperature, the conditions with a reaction temperature of 10°C to 80°C, preferably 20°C to 50°C, and more preferably 30°C to 40°C can be employed. As for the reaction temperature, 37°C is exemplified, for example. The reaction time is also not particularly limited, and may be suitably set depending on various conditions. The reaction time may be, for example, 5 minutes to 2 hours, preferably 15 minutes to 90 minutes, and more preferably 30 minutes to 40 minutes.

### <Method for measuring endotoxin>

Another aspect of the present invention is an endotoxin measuring method in which sensitivity of an endotoxin measuring reagent to the endotoxin of Helicobacter pylori is enhanced, in which the method includes preparing an endotoxin measurement sample containing the endotoxin measuring reagent and an analyte, the endotoxin measuring reagent contains a recombinant protein of horseshoe crab factor C, and enhancement of the sensitivity is achieved by increasing the concentration of the recombinant protein of factor C in the endotoxin measurement sample (hereinbelow, the endotoxin measuring method may be referred to as a "measurement method of the present invention"). Another embodiment of the present invention is a method for measuring endotoxin in which the method includes preparing an endotoxin measurement sample containing an endotoxin measuring reagent and an analyte, the endotoxin measuring reagent contains a recombinant protein of horseshoe crab factor C, and the concentration of the recombinant protein of factor C in the endotoxin measurement sample is increased to an amount that is sufficient for enhancing the sensitivity of the endotoxin measuring reagent to the endotoxin of Helicobacter pylori.

In the present invention, the measurement is used as a general description including detection, sensing, and quantification. Thus, the measurement method of the present invention may be a method for detecting endotoxin, a method for sensing endotoxin, or a method for quantifying endotoxin, for example.

The measurement method of the present invention is an endotoxin measuring method in which sensitivity of an endotoxin measuring reagent to the endotoxin of Helicobacter pylori is enhanced, in which the sensitivity is enhanced by increasing the concentration of a recombinant protein of factor C in an endotoxin measurement sample. With regard to the measurement method of the present invention, the method can be carried out under well-known conditions for measuring endotoxin except that the concentration of the recombinant protein of factor C is increased in an endotoxin measurement sample. The aspect of the concentration amount of the recombinant protein of factor C in an endotoxin measurement sample, which is sufficient for enhancing the sensitivity of an endotoxin measuring reagent to the endotoxin of Helicobacter pylori, is the same as the aspect of the content of the recombinant protein of factor C described in <Method for enhancing sensitivity of endotoxin measuring reagent> above.

Hereinbelow, details of the measurement method are described.

The measurement method of the present invention is an endotoxin measuring method including the following steps (A) and (B):
(A) a step of mixing a recombinant protein of horseshoe crab factor C and an analyte; and
(b) a step of measuring protease activity of Limulus factor.

The above step (A) is a step of mixing a recombinant protein of horseshoe crab factor C and an analyte. In a case in which the analyte is a sample containing endotoxin, a factor C contacted with the endotoxin converts into an active form factor C.

With regard to the above step (A), as long as an operation of mixing a recombinant protein of factor C and an analyte is included, it may further include an operation of mixing other materials. Examples of "other materials" described herein include a factor B, a pro-clotting enzyme, and a compound for detection (substrate for detection). In a case in which the above step (A) is a step of mixing a recombinant protein of factor C, a factor B, a pro-clotting enzyme, and an analyte and also the analyte is a sample containing endotoxin, a cascade reaction by which a factor C brought into contact with the endotoxin converts into an active form factor C, a factor B converts into an active form factor B, and a pro-clotting enzyme converts into a clotting enzyme progresses.

In the present invention, the "factor C" is not particularly limited as long as it is a factor C having the function of a horseshoe crab factor C. Definition of the "horseshoe crab" described herein is the same as the description given in <Method for enhancing sensitivity of endotoxin measuring reagent> described above. The expression of "function of a horseshoe crab factor C" means the function of a horseshoe crab factor C as a protease precursor. The expression "function of a horseshoe crab factor C" specifically means the function of conversion into an active form (active form factor C) in the co-existence of endotoxin and expression of the protease activity. The function of a horseshoe crab factor C is a function of conversion into an active form (active form factor C) in the co-existence of endotoxin and conversion into an active form (active form factor B) by cleaving off the factor B, for example. Furthermore, the function of a horseshoe crab factor C is a function of conversion into an active form (active form factor C) in the co-existence of endotoxin and release of a labeling material by cleaving off a compound for detection, which becomes a substrate of the active form factor C, for example. As for the compound for detection described herein, the aspect described in the followings can be suitably used, for example.

In the present invention, the "factor B" is not particularly limited as long as it is a factor B having the function of a horseshoe crab factor B. Definition of the "horseshoe crab" described herein is the same as the description given in <Method for enhancing sensitivity of endotoxin measuring reagent> described above. The expression of "function of a horseshoe crab factor B" specifically means the function of conversion into an active form (active form factor B) in accordance with a contact with an active form factor C and expression of the protease activity. The function of a horseshoe crab factor B is a function of conversion into an active form (active form factor B) in accordance with a contact with an active form factor C and conversion into a clotting enzyme by cleaving off a pro-clotting enzyme, for example. Furthermore, the function of a horseshoe crab factor B is a function of conversion into an active form (active form factor B) in accordance with a contact with an active form factor C and release of a labeling material by cleaving off a compound for detection, which becomes a substrate of the active form factor B, for example. As for the compound for detection described herein, the aspect described in the followings can be suitably used, for example.

The "pro-clotting enzyme" described in the present invention is not particularly limited as long as it is a pro-clotting enzyme having the function of a horseshoe crab pro-clotting enzyme. Definition of the "horseshoe crab" described herein is the same as the description given in <Method for enhancing sensitivity of endotoxin measuring reagent> described above. The expression of "function of a horseshoe crab pro-clotting enzyme" means the function of a horseshoe crab pro-clotting enzyme as a protease precursor. The expression of "function of a horseshoe crab pro-clotting enzyme" specifically means the function of conversion into an active form (clotting enzyme) in the co-existence of an active form factor B and expression of the protease activity. The function of a horseshoe crab pro-clotting enzyme indicates a function of conversion into an active form (clotting enzyme) in the co-existence of an active form factor B and forming of coagulin gel by cleaving off coagulogen, for example. Furthermore, the function of a horseshoe crab pro-clotting enzyme is a function of conversion into an active form (clotting enzyme) in the co-existence of an active form factor B and release of a labeling material by cleaving off a compound for detection, which becomes a substrate of the clotting enzyme, for example. As for the compound for detection described herein, the aspect described in the followings can be suitably used, for example.

As for the factor C of the present invention, specifically, the polypeptide expressed in any one of the following (1) to (4) is exemplified.
(1) Polypeptide having the amino acid sequence represented by SEQ ID NO: 2.
(2) Polypeptide having the amino acid sequence represented by SEQ ID NO: 4.
(3) Polypeptide having the amino acid sequence represented by SEQ ID NO: 12.
(4) Polypeptide having an amino acid sequence in which one or plural amino acid residues of the amino acid sequence shown in any one of the above (1) to (3) are substituted, deleted, inserted, and/or added, and also having the function of a horseshoe crab factor C.
   As for the factor B of the present invention, specifically, the polypeptide expressed in any one of the following (5) and (6) is exemplified.
(5) Polypeptide having the amino acid sequence represented by SEQ ID NO: 6.
(6) Polypeptide having an amino acid sequence in which one or plural amino acid residues of the amino acid sequence shown in the above (5) are substituted, deleted, inserted, and/or added, and also having the function of a horseshoe crab factor B.
   As for the pro-clotting enzyme of the present invention, specifically, the polypeptide expressed in any one of the following (7) and (8) is exemplified.
(7) Polypeptide having the amino acid sequence represented by SEQ ID NO: 8.
(8) Polypeptide having an amino acid sequence in which one or plural amino acid residues of the amino acid sequence shown in the above (7) are substituted, deleted, inserted, and/or added, and also having the function of a horseshoe crab pro-clotting enzyme.

The expression "plural" described in the above (4), (6), and (8) means the number (total number) of amino acid residues that are substituted, deleted, inserted, and/or added to the extent that allows no functional loss of the polypeptides as Limulus factor. The expression "plural" may be the number of preferably 10% or less, more preferably 5% or less, even more preferably 2% or less, particularly preferably 1% or less, and most preferably 0.5% or less relative to the total number of amino acid residues constituting the polypeptide, for example.

Accordingly, since the total number of amino acid residues is 1,019 in the case of the amino acid sequence of the polypeptide shown in the above (4), the "plural" may be preferably 2 to 100, more preferably 2 to 50, even more preferably 2 to 20, particularly preferably 2 to 10, and most preferably 2 to 5. The expression "plural" described in the above (4), (6), and (8) may be, as a specific individual number, an integer of 2, 3, 4, 5, or the like.

The expression "substituted, deleted, inserted, and/or added" described in the above (4), (6), and (8) indicates a conservative mutation, for example. Representative example of the conservative mutation is a conservative substitution. The conservative substitution means a mutation for having a substitution among Phe, Trp, and Tyr when an aromatic amino acid is present at the substitution site, a substitution among Leu, Ile, and Val when a hydrophobic amino acid is present at the substitution site, a substitution between Gln and Asn when a polar amino acid is present at the substitution site, a substitution among Lys, Arg, and His when a basic amino acid is present at the substitution site, a substitution between Asp and Glu when an acidic amino acid is present at the substitution site, or a substitution between Ser and Thr when an amino acid having hydroxyl group is present at the substitution site. Specific examples of the substitution which is regarded as conservative mutation includes a substitution of Ser or Thr for Ala, a substitution of Gln, His, or Lys for Arg, a substitution of Glu, Gln, Lys, His, or Asp for Asn, a substitution of Asn, Glu, or Gln for Asp, a substitution of Ser or Ala for Cys, a substitution of Asn, Glu, Lys, His, Asp, or Arg for Gln, a substitution of Gly, Asn, Gln, Lys, or Asp for Glu, a substitution of Pro for Gly, a substitution of Asn, Lys, Gln, Arg, or Tyr for His, a substitution of Leu, Met, Val, or Phe for Ile, a substitution of Ile, Met, Val, or Phe for Leu, a substitution of Asn, Glu, Gln, His, or Arg for Lys, a substitution of Ile, Leu, Val, or Phe for Met, a substitution of Trp, Tyr, Met, Ile, or Leu for Phe, a substitution of Thr or Ala for Ser, a substitution of Ser or Ala for Thr, a substitution of Phe or Tyr for Trp, a substitution of His, Phe, or Trp for Tyr, and a substitution of Met, Ile, or Leu for Val.

Each of the polypeptides described in the above (4), (6), and (8) has similarity of preferably 85% or higher, more preferably 90% or higher, even more preferably 95% or higher, and particularly preferably 99% or higher to the amino acid sequences of the polypeptides described in the above (1), (2), (3), (5), and (7), for example, and each of the polypeptides may be also a polypeptide having the function of Limulus factor. Furthermore, the "similarity" described herein is a concept which includes "identity", and thus an application can be made to a suitable aspect of the polypeptide by interchanging the similarity with identity.

The above polypeptides can be prepared by a well-known method on the basis of the descriptions of the present specification. For example, the above polypeptides can be prepared by genetic engineering techniques. Specifically, the polypeptides can be obtained by artificially introducing a DNA encoding the above polypeptide to host cells and expressing the polypeptide therein.

Examples of the DNA encoding the above polypeptides include the followings.
[1] DNA having the base sequence represented by SEQ ID NO: 1.
[2] DNA having the base sequence represented by SEQ ID NO: 3.
[3] DNA having the base sequence represented by SEQ ID NO: 5.
[4] DNA having the base sequence represented by SEQ ID NO: 7.
[5] DNA having the base sequence represented by SEQ ID NO: 9.
[6] DNA having the base sequence represented by SEQ ID NO: 10.
[7] DNA having the base sequence represented by SEQ ID NO: 11.

The above polypeptides may be prepared as a culture solution itself obtained by culturing cells, or may be prepared as a fraction obtained by purifying the culture solution to desired extent, for example.

The "compound for detection" described in the present invention is a substrate which is used for measuring the presence or absence or the amount of activated Limulus factor or progress of the cascade reaction. The compound for detection may be a substrate for measuring an active form factor C, a substrate for measuring an active form factor B, or a substrate for measuring a clotting enzyme. The compound for detection is not particularly limited as long as it is a substrate of activated Limulus factor. The compound for detection may be either a protein, a peptide, or a derivative thereof, for example.

The protein may be either a natural protein or a recombinant protein. As for the protein, coagulogen as a substrate of the clotting enzyme is exemplified. The natural coagulogen can be prepared by fractionation from a lysate, for example. Furthermore, the recombinant coagulogen can be prepared in view of the method described in the literature (Miyata et al., Separate Issue of Proteins, Nucleic acids, and Enzymes, No. 29; P30-43; 1986), for example.

The peptide may be a synthetic substrate obtained by chemical synthesis, for example. The synthetic substrate is not particularly limited as long as it is a substrate suitable for measuring the presence or absence or the amount of activated Limulus factor or the progress of the cascade reaction. The synthetic substrate is preferably a peptide derivative.

As for the synthetic substrate, a substrate represented by the general formula Y-X-Z (in the formula, Y may or may not be present, and, when Y is present, Y is a protecting group (namely, Y is a hydrogen atom or a protecting group), X is a peptide, and Z is a labeling material) is exemplified. It is preferable that this synthetic substrate has a property of cleaving off the covalent bond between X and Z by activated Limulus factor and releasing the labeling material Z. In the above general formula, Y is preferably a protecting group for the amino group at the N terminal of a peptide. In the above general formula, the bond between Y and X is preferably an amide bond which is formed between the carboxy group of a protecting group and an α amino group at the N terminal of a peptide. Furthermore, in the above general formula, the bond between X and Z is preferably an amide bond which is formed between the carboxy group at the C terminal of a peptide and an amino group of the labeling material Z.

The protecting group as Y is not particularly limited, and a well-known protecting group which is applicable for peptide protection can be used. Examples of the protecting group include a tert-butoxycarbonyl group (Boc), a benzyloxycarbonyl group (Cbz), a benzyl group (Bzl), a benzoyl group (Bz), and an acetyl group (Ac).

The peptide (X) is not particularly limited as long as it is a peptide having an amino acid sequence that is a substrate of activated Limulus factor. The peptide is preferably a substrate that is suitable for measuring serine protease, and is preferably a peptide having an Arg (R) residue at the C terminal.

In a case in which the activated Limulus factor is a factor C, the peptide is preferably a peptide having an amino acid sequence represented by the general formula X-Pro-Arg (in the formula, X represents an arbitrary amino acid). Specifically, in a case in which the activated Limulus factor is a factor C, the peptide is preferably a peptide having an amino acid sequence represented by Val-Pro-Arg (VPR) or Asp-Pro-Arg (DPR).

In a case in which the activated Limulus factor is a factor B, the peptide is preferably a peptide having an amino acid sequence represented by the general formula X-Thr-Arg (in the formula, X represents an arbitrary amino acid). Specifically, in a case in which the activated Limulus factor is a factor B, the peptide is preferably a peptide having an amino acid sequence represented by Leu-Thr-Arg (LTR) or Met-Thr-Arg (MTR).

In a case in which the activated Limulus factor is a pro-clotting enzyme, the peptide is preferably a peptide having an amino acid sequence represented by the general formula X-Gly-Arg (in the formula, X represents an arbitrary amino acid). Specifically, in a case in which the activated Limulus factor is a pro-clotting enzyme, the peptide is preferably a peptide having an amino acid sequence represented by Leu-Gly-Arg (LGR) or Glu-Gly-Arg (EGR) .

The labeling material (Z) is not particularly limited, and a well-known labeling material applicable for measurement of protease activity can be used. As a labeling material, a compound allowing optical detection (of color, fluorescence, light emission, or the like) upon release from a peptide can be used, for example. Examples of such labeling material include para-nitroaniline (pNA), 7-methoxycoumarine-4-acetic acid, 2,4-dinitroaniline (DNP), 7-amino-4-methylcoumarin (AMC), and 7-amino-4-trifluoromethyl coumarin. Furthermore, as a labeling material, a compound allowing detection by an electrochemical measurement method (voltammetry, amperometry, or the like) upon release from a peptide can be used, for example. Examples of such a labeling material include p-aminophenol (pAP), p-methoxyaniline (pMA), N-methyl-p-phenylenediamine (MPDD), and N,N'-dimethyl-p-phenylenediamine (DMPD).

In the step (A) of the above measurement method of the present invention, the Limulus factor, the analyte, the compound for detection, and other materials (buffering reagent or the like) may be added at any order and mixed with one another. For example, in the above step (A), the analyte may be added to a mixture of the Limulus factor, the compound for detection, and other materials and mixed therein. Furthermore, for example, in the above step (A), a mixture of the Limulus factor, the compound for detection, and other materials may be added to the analyte and mixed therein. Mixing in the step (A) may be carried out inside of a vessel (in a vessel) having an opening on one end (test tube, vial, or the like), for example. The analyte is not particularly limited as long as it is a sample from which endotoxin detection is required, and examples thereof include, in addition to water for injection, pharmaceutical product, transfusion solution, blood preparation, medical device (medical instrument), quasi drug, cosmetic product or the like, food product, environmental sample such as water, air, river, or soil, natural protein, gene recombinant protein, nucleic acid, enzyme, carbohydrate, and electrolyte, components of a living body like blood, body fluid, and tissues.

The above step (B) is a step of measuring protease activity of the polypeptide to be used in the present invention. This step is a step of measuring a labeling material released from a compound for detection, for example. During this step, the labeling material is released, in an amount (mole number) according to the protease activity (total activity) of an active form Limulus factor, from a compound for detection, and therefore protease activity of the polypeptide to be used in the present invention can be measured by measuring the labeling material released from a compound for detection. The labeling material released from a compound for detection can be measured, for example, by a photometric device such as a spectrophotometer or a fluorometer. Furthermore, the labeling material released from a compound for detection can be measured, for example, by an electrochemical measurement device such as a voltammetric device or an amperometric device. For example, by comparing the measurement value shown from the above step with a blank value (measurement value obtained by having an endotoxin-free analyte as a measurement subject), the presence or absence of endotoxin in an analyte can be determined. Furthermore, the measurement method of the present invention may be a step for determining the presence or absence of endotoxin in an analyte by determining the presence or absence of gelation of a mixture solution, for example.

The measurement method of the present invention may include another step in addition to the above steps (A) and (B). The measurement method of the present invention may include a step for determining the presence or absence of endotoxin in an analyte by comparing the measurement value obtained from the step (B) with a blank value. Furthermore, the measurement method of the present invention may include a step for converting the measurement value obtained from the step (B) into other value, for example. As a step for converting the measurement value into other value, a step of calculating the amount of endotoxin on the basis of measurement value is exemplified, for example. Specifically, this step is a step of converting the measurement value that is obtained from measurement of an analyte into the amount of endotoxin on the basis of relationship (standard curve) between the measurement value obtained from measurement by replacing an analyte with a standard material with known concentration and the concentration of standard material, for example.

The Limulus reaction in the measurement method of the present invention is preferably carried out in an aqueous solvent.

### <Endotoxin measuring reagent>

In another aspect of the present invention, there is provided an endotoxin measuring reagent containing a horseshoe crab factor C. The horseshoe crab factor C contained in the endotoxin measuring reagent according to the present invention is the following (a) and may further contain (b) and/or (c), and the present invention may also include an aspect of the endotoxin measuring reagent in which relative potency of the endotoxin of Helicobacter pylori is 0.1 times or more relative potency measured by using a lysate reagent:
(a) a recombinant protein of horseshoe crab factor C;
(b) a recombinant protein of horseshoe crab factor B; and
(c) a recombinant protein of horseshoe crab pro-clotting enzyme.

Herein, the aspect of the recombinant protein of the Limulus factor of (a), (b), and (c) is the same as the aspect of <Method for measuring endotoxin> described above.

The lysate reagent may be, for example, the lysate reagent mentioned above in <Method for enhancing sensitivity of endotoxin measuring reagent> described above.

The endotoxin measuring reagent can be suitably used for carrying out the measurement method of the present invention.

As it has been described in the above, when the relative potency of the endotoxin of Helicobacter pylori measured by using an endotoxin measuring reagent does not correspond to desired potency, the relative potency of the endotoxin of Helicobacter pylori can be enhanced by increasing the content of the recombinant protein of factor C at the time of endotoxin measurement.

As another aspect, the present invention includes at least the above (a) and may further include (b) and/or (c), and the endotoxin measuring reagent can be an endotoxin measuring reagent in which relative potency (measured by a colorimetric method, a fluorescence method, or the like) of the endotoxin of Helicobacter pylori is 200 (EU/µg) or higher, preferably 250 (EU/µg) or higher, and more preferably 300 (EU/µg) or higher.

Herein, the aspect of the recombinant protein of the Limulus factor of (a), (b), and (c) is the same as the aspect of <Method for measuring endotoxin> described above.

The relative potency of the endotoxin of Helicobacter pylori may be, for example, 200 (EU/ug) or higher and 3,000 (EU/µg) or lower, 250 (EU/ug) or higher and 2,500 (EU/µg) or lower, or 300 (EU/µg) or higher and 2,000 (EU/pg) or lower. In an embodiment, the endotoxin measuring reagent contains a recombinant protein of factor C in an amount at which the relative activity is achieved. The relative potency of the endotoxin of Helicobacter pylori is a value measured using a measurement sample containing the endotoxin of Helicobacter pylori at a concentration equal to or more than the lower limit of quantitation.

Furthermore, the present aspect may be a specific example of the aspect in which the relative potency of the endotoxin of Helicobacter pylori is 0.1 times or more the relative potency measured with the lysate reagent.

The endotoxin measuring reagent may contain other configurations as long as the above components are contained as a constitutional component. Examples of other configurations described herein include a compound for detection, an instruction for use describing the product information or the like. The instruction for use may clearly describe that, when the relative potency of the endotoxin of Helicobacter pylori of an endotoxin measuring reagent does not correspond to the desired potency, the content of the recombinant protein of factor C at the time of endotoxin measurement is increased to an amount that is sufficient for enhancement of the sensitivity.

In an embodiment, the endotoxin measuring reagent is based on a three factor system and contains a compound for detection represented by the above general formula Y-X-Z (in the formula, Y is a hydrogen atom or a protecting group, X is a peptide containing the amino acid sequence, which is a substrate of a pro-clotting enzyme, and Z becomes a labeling material which is optically detectable when being released from X). Examples of Z may include para-nitroaniline, 7-methoxycoumarine-4-acetic acid, 7-amino-4-methylcoumarin, and 7-amino-4-trifluoromethyl coumarin. In a specific embodiment, Z is a labeling material, which is detectable as coloring when being released from X, and is preferably para-nitroaniline.

In an embodiment, the endotoxin measuring reagent is based on a two factor system and contains a compound for detection represented by the above general formula Y-X-Z (in the formula, Y is a hydrogen atom or a protecting group, X is a peptide containing the amino acid sequence, which is a substrate of a factor B, and Z becomes a labeling material which is optically detectable when being released from X). Examples of Z may include para-nitroaniline, 7-methoxycoumarine-4-acetic acid, 7-amino-4-methylcoumarin, and 7-amino-4-trifluoromethyl coumarin. In a specific embodiment, Z is a labeling material, which is detectable as fluorescence when being released from X, and is preferably 7-methoxycoumarine-4-acetic acid, 7-amino-4-methylcoumarin, or 7-amino-4-trifluoromethyl coumarin.

In an embodiment, the endotoxin measuring reagent is based on a one factor system and contains a compound for detection represented by the above general formula Y-X-Z (in the formula, Y is a hydrogen atom or a protecting group, X is a peptide containing the amino acid sequence, which is a substrate of a factor C, and Z becomes a labeling material which is optically detectable when being released from X). Examples of Z may include para-nitroaniline, 7-methoxycoumarine-4-acetic acid, 7-amino-4-methylcoumarin, and 7-amino-4-trifluoromethyl coumarin. In a specific embodiment, Z is a labeling material, which is detectable as fluorescence when being released from X, and is preferably 7-methoxycoumarine-4-acetic acid, 7-amino-4-methylcoumarin, or 7-amino-4-trifluoromethyl coumarin. Furthermore, in the case of a one factor system, as compared to a two factor system or a three factor system, the relative potency of 200 EU/µg or higher of the endotoxin of Helicobacter pylori can be achieved even with a small amount of the recombinant protein of factor C.

The Limulus factor in the above endotoxin measuring reagent is preferably provided as a freeze-dried product.

One aspect of the present invention relates to an endotoxin measuring reagent having enhanced sensitivity to the endotoxin of Helicobacter pylori.

Another aspect of the present invention is a method for producing an endotoxin measuring reagent having enhanced sensitivity to the endotoxin of Helicobacter pylori, in which the endotoxin measuring reagent contains a recombinant protein of horseshoe crab factor C and includes increasing the content of the recombinant protein of factor C in the endotoxin measuring reagent (hereinbelow, the method may be referred to as a "production method of the present invention"). According to an embodiment of the present invention, the content of the recombinant protein of factor C in the endotoxin measuring reagent is increased to an amount that is sufficient for enhancing the sensitivity.

With regard to the aspect relating to the content of the recombinant protein of factor C in the endotoxin measuring reagent that is sufficient for enhancement of the sensitivity, for example, the content can be set so as to be an aspect of the content of the recombinant protein factor C in a measurement sample at the time of endotoxin measurement described in <Method for enhancing sensitivity of endotoxin measuring reagent> above. Specifically, the content can be varied depending on desired relative potency, measurement system, hosts of the recombinant protein of factor C, or the like. In a case in which the measurement system is based on three factor system, although it is not limited, the content of the recombinant protein of factor C in an endotoxin measuring reagent (in an amount of single use) can be set, in terms of the concentration in a measurement sample at the time of endotoxin measurement, at 140 ng/mL or more, 162 ng/mL or more, 180 ng/mL or more, 200 ng/mL or more, 250 ng/mL or more, 300 ng/mL or more, 400 ng/mL or more, 500 ng/mL or more, 600 ng/mL or more, or 700 ng/mL or more. In a case in which the measurement system is a two factor system, although it is not limited, the content of the recombinant protein of factor C in an endotoxin measuring reagent (in an amount of single use) can be set, in terms of the concentration in a measurement sample at the time of endotoxin measurement, at 235 ng/mL or more, 250 ng/mL or more, 300 ng/mL or more, or 350 ng/mL or more as the concentration at the time of endotoxin measurement. In a case in which the measurement system is a one factor system, although it is not limited, the content of the recombinant protein of factor C in an endotoxin measuring reagent (in an amount of single use) can be set, in terms of the concentration in a measurement sample at the time of endotoxin measurement, at 10 ng/mL or more, 20 ng/mL or more, 30 ng/mL or more, 40 ng/mL or more, 50 ng/mL or more, 60 ng/mL or more, 70 ng/mL or more, 100 ng/mL or more, 500 ng/mL or more, or 800 ng/mL or more.

The upper limit of the content of the recombinant protein of factor C in the measurement sample at the time of endotoxin measurement is not particularly limited, and is, for example, 10 mg/mL or less and is preferably less than 10 ug/mL (for example, 5 ug/mL or less or 2 ug/mL or less) from the viewpoint of production efficiency.

The measuring reagent of the present invention and the endotoxin measuring reagent produced by the production method of the present invention can be suitably used for carrying out the measurement method of the present invention.

As the content of the recombinant protein of factor C is increased in the measuring reagent of the present invention and the endotoxin measuring reagent produced by the production method of the present invention, the relative potency of the endotoxin of Helicobacter pylori measured by using the endotoxin measuring reagent is enhanced.

The measuring reagent of the present invention and the endotoxin measuring reagent produced by the production method of the present invention may further contain other configurations as long as a recombinant protein of factor C is contained therein as a constitutional component. Examples of other configurations described herein include a factor B, a pro-clotting enzyme, a compound for detection, and an instruction for use describing the product information. The aspect of the recombinant protein of factor C and the above other constitutional components are the same as the aspect of the recombinant protein of factor C and other configurations that are described in <Method for enhancing sensitivity of endotoxin measuring reagent> and <Method for measuring endotoxin> above. In an embodiment, the endotoxin measuring reagent does not substantially contain a surfactant.

According to the production method of the present invention, the production can be made by a well-known method for producing a recombinant reagent except that the content of the recombinant protein of factor C is increased in the endotoxin measuring reagent. Namely, by combining a recombinant protein of factor C with the above other constitutional components, the endotoxin measuring reagent can be produced.

### <Kit for endotoxin measurement>

Furthermore, the present invention may also include an aspect of a kit for endotoxin measurement that is characterized by including the above endotoxin measuring reagent as a constitutional product.

The kit can be suitably used for carrying out the measurement method of the present invention.

Furthermore, the kit may further include other constitutional products as long as it includes the above endotoxin measuring reagent as a constitutional product. Examples of other constitutional products described herein include a compound for detection, a buffering reagent, distilled water, Reference Standard Endotoxin, a micro plate, and an instruction for use describing the product information.

The kit may contain each constitutional product that is separately present, or may contain a mixture obtained by combining arbitrarily each constitutional product. For example, the kit may contain each Limulus factor that is separately present, may contain Limulus factors according to an aspect in which Limulus factors have been mixed in advance, or may further contain a compound for detection according to an aspect in which the compound has been mixed in advance.

### Examples

Hereinbelow, the present invention is specifically described in view of Examples, but these are merely exemplifications of the present invention and the scope of the present invention is not limited thereto.

### <Example 1: Comparison of sensitivity to endotoxin among reagents>

By employing a recombinant reagent and a lysate reagent, comparison was made to see any difference in the sensitivity to endotoxins of different origin.

The following endotoxin measuring reagents were prepared. Furthermore, the "recombinant reagent" indicates a preparation which contains a factor C produced by genetic recombination techniques, and the "lysate reagent" indicates a preparation which contains a natural factor C prepared from hemocyte extract components of a horseshoe crab.
(1) Preparation containing recombinant factor C (rFC), recombinant factor B (rFB), recombinant pro-clotting enzyme (rPCE), compound for detection (synthetic substrate Boc-LGR-pNA; PEPTIDE INSTITUTE, INC.) and additives of PyroSmart (registered trademark): commercially available recombinant reagent A
(2) PyroGene (LONZA JAPAN): commercially available recombinant reagent B
(3) EndoZyme (registered trademark) II (bioMérieux Japan Ltd.): commercially available recombinant reagent C
(4) Endospecy (registered trademark) ES-50M (SEIKAGAKU CORPORATION): commercially available lysate reagent A
(5) Kinetic QCL (LONZA JAPAN): commercially available lysate reagent B

As an endotoxin to be measured, endotoxins of two types, Helicobacter pylori CA2 (LPS Research Institute) and Salmonella minnesota R595Re (List Biological Laboratories, Inc.), were used. Furthermore, as Reference Standard Endotoxin(RSE) derived from Escherichia coli O113: H10, Reference Standard Endotoxin of Japanese Pharmacopoeia (Pharmaceutical and Medical Device Regulatory Science Society of Japan) or US Pharmacopoeia (SEIKAGAKU CORPORATION) was used.

Endotoxin of Helicobacter pylori CA2 was dissolved in water for injection (Otsuka Pharmaceutical Factory, Inc.) to have 1 mg/mL. Endotoxin of Salmonella minnesota R595Re was dissolved to have 1 mg/mL using 0.1% (v/v) triethylamine (FUJIFILM Wako Pure Chemical Corporation), and then neutralized with Tris buffer solution (FUJIFILM Wako Pure Chemical Corporation). The stock solution of those endotoxins was diluted with water for injection such that the endotoxin concentration falls within the quantification range described in the instruction for use for each reagent, and thus a diluted solution of endotoxin was obtained.

For the measurement using the commercially available recombinant reagent A, the preparation was mixed, in a micro plate, with water for injection (blank) or a diluted solution of endotoxin to prepare a measurement sample. According to this assay system, a factor C (FC) is activated by endotoxin to produce an active form FC, a factor B (FB) is activated by the active form FC to produce an active form FB, a pro-clotting enzyme (PCE) is activated by the active form FB to produce a clotting enzyme, and synthetic substrate Boc-LGR-pNA is cleaved off by the clotting enzyme to release para-nitroaniline. By measuring the absorbance of a measurement sample using an absorbance microplate reader (for 30 minutes at 37°C, with a main wavelength of 405 nm and a reference wavelength of 492 nm and an interval of 15 seconds), the absorbance change ratio per unit time (for 1 minute) was calculated (mAbs/min). Subsequently, from a calibration curve established with RSE, endotoxin activity (EU/mL) of a diluted solution of endotoxin at each concentration was calculated, and, by dividing the calculated value by each concentration of diluted solution of endotoxin, relative potency (EU/µg) at each endotoxin concentration was calculated. Among the obtained relative potencies, values falling within the quantification range of RSE were averaged to determine the relative potency of endotoxin (Table 1).

For other recombinant reagents (commercially available recombinant reagent B, commercially available recombinant reagent C) and lysate reagents (commercially available lysate reagent A, commercially available lysate reagent B), the relative potency was also determined in the same manner as that for the commercially available recombinant reagent A on the basis of the results measured according to an instruction for use (Table 1).

As a result, the relative potency of endotoxin of Helicobacter pylori CA2, which has been obtained from the commercially available recombinant reagents A, B, and C, was 147.3, 3.21, and 0.07 EU/ug, respectively. Those values were significantly lower than the relative potency obtained by using a lysate reagent (about 2,000 EU/µg), and the difference was 14 times or more (Table 1). Namely, a huge difference was seen in terms of the sensitivity to endotoxin of Helicobacter pylori CA2 between the commercially available recombinant reagent and lysate reagent. On the other hand, the relative potency of endotoxin of Salmonella minnesota R595Re showed no huge difference between the commercially available recombinant reagent and lysate reagent (Table 1).

Furthermore, the same test as above was carried out by using endotoxin of Escherichia coli O55: B5 and Salmonella typhimulium. However, the relative potency of those endotoxins also showed no huge difference between the commercially available recombinant reagent and lysate reagent.

**Table 1**

| | Relative potency (EU/µg) when RSE is taken as reference | |
|---|---|---|
| | H. pylori | S. minnesota |
| Commercially available recombinant reagent A | 147.3 | 54448 |
| Commercially available recombinant reagent B | 3.21 | 60027 |
| Commercially available recombinant reagent C | 0.07 | 28329 |
| Commercially available lysate reagent A | 2193 | 43240 |
| Commercially available lysate reagent B | 1971 | 54484 |

### <Example 2: Influence of rFC content on sensitivity to endotoxin of Helicobacter pylori CA2>

### A. Preparation of recombinant protein

According to Mizumura H, Ogura N, Aketagawa J, Aizawa M, Kobayashi Y, Kawabata SI, Oda T. Innate Immun. 2017 Feb; 23 (2): 136-146, or "Recombinant proteins derived from limulus bacterium, and DNA molecules encoding same" (WO 2018/074498 A1), rFC, rFB, and rPCE of Tachypleus tridentatus (T.t.) were expressed as described below to obtain various kinds of enzyme solution. Furthermore, SEQ ID NO: 1 was used as DNA encoding FC of T.t., SEQ ID NO: 5 or 9 was used as DNA encoding FB of T.t., and SEQ ID NO: 7 was used as DNA encoding PCE of T.t.

### (1) Stable expression of rFC using mammalian cells (CHO DG44 cells)

Chinese hamster ovarian cell line (CHO DG44 cells) was used as a host, and an enzyme solution of rFC was prepared according to the following procedure.

DNA (SEQ ID NO: 1) encoding FC of T.t. was inserted between the EcoRI and XbaI recognition sites of an expression vector (pCI-neo; manufactured by Promega Corporation) to produce a plasmid for FC expression. The plasmid for FC expression and a plasmid for expressing dehydrofolate reductase (dhfr) were introduced to CHO DG44 cells by using a lipofection reagent (Lipofectamine LTX; Life Technologies Corporation). The cells were subjected to static culture under supply of 5 (v/v)% CO₂ at 37°C, and the cells having the expression plasmid introduced onto the genome were selected with geneticin (Invitrogen) to obtain a cell line highly expressing rFC from the poly clones. From polyclonal cell group which has been acclimated in a medium containing 5 µM methotrexate (MTX), the cell line highly expressing rFC was monoclonized. The cell line (mono clone) was floated after undergoing acclimation in serum-free complete synthetic medium. From the floating culture solution in which cells were grown to later stage of logarithmic growth phase under supply of 5 (v/v)% CO₂ at 37°C, culture supernatant was obtained by centrifugation (3,000 ×g, for 30 minutes, 4°C) to prepare an enzyme solution of rFC.

### (2) Stable expression of rFC using insect cells (Sf9 cells)

An enzyme solution of rFC was prepared according to the following procedure, by using insect cells (Sf9 cells) as host cells.

DNA (SEQ ID NO: 1) encoding FC of T.t. was inserted between the EcoRV and MluI recognition sites of an expression vector (pIZ-V5; Life Technologies Corporation) to produce a plasmid for FC expression. The plasmid for FC expression was introduced to Sf9 cells by using a cellfectin reagent (Life Technologies Corporation). The cells were subjected to static culture at 28°C, and the cells having the expression plasmid introduced onto the genome were selected against zeocin (Invitrogen). After that, candidates in which the factor C gene is stably expressed were isolated by a cloning cylinder method, and Sf9 cell line highly expressing rFC was selected. The Sf9 cell line highly expressing rFC was then subjected to floating culture (28°C) to later stage of logarithmic growth phase (6 to 8 × 10⁶ cells/mL) in Sf900III medium containing 1x penicillin/streptomycin (Life Technologies Corporation) and 50 ug/mL zeocin. Culture supernatant was obtained by centrifugation (3,000 ×g, for 30 minutes, 4°C) of the culture solution to prepare an enzyme solution of rFC.

### (3) Stable expression of rFB and rPCE using insect cells (Sf9 cells)

Insect cells (Sf9 cells) were used as a host, and enzyme solutions of rFB and rPCE were prepared according to the following procedure.

DNA (SEQ ID NO: 7, 9) encoding FB and PCE of T.t. was inserted between the EcoRV and MluI recognition sites of an expression vector (pIZ-V5; Life Technologies Corporation) to produce each of a plasmid for FB expression and a plasmid for PCE expression. Furthermore, the base sequence represented by SEQ ID NO: 9 is a base sequence in which the base sequence represented by SEQ ID NO: 5 is optimized for expression in insect cells. According to the same method as FC above, enzyme solutions of rFB and rPCE were prepared. The protein concentration in the enzyme solutions of rFB and rPCE was measured by using a protein assay kit (Bio-Rad Laboratories, Inc.).

### (4) Transient expression of rFC using Expi CHO Expression System

By using Expi CHO Expression System (Thermo Fisher Scientific Inc.), an enzyme solution of rFC was prepared according to the attached instructions.

DNA (SEQ ID NO: 1) encoding FC of T.t. was inserted between the EcoRI and XbaI recognition sites of an expression vector (pCI-neo; manufactured by Promega Corporation) to produce a plasmid for FC expression. The plasmid for FC expression which has been mixed with ExpiFectamin CHO was introduced to Expi CHO-S cells in the co-existence of Expi CHO Expression medium. The cells were grown in serum-free complete synthetic medium under supply of 8 (v/v)% CO₂ at 37°C. The floating culture solution was subjected to centrifugation (3,000 ×g, for 30 minutes, 4°C) to obtain culture supernatant, and thus an enzyme solution of rFC was prepared.

### B. Measurement of rFC concentration

The rFC concentration in a sample was calculated by ELISA method using anti FC antibody in view of Kobayashi Y, Shiga T, Shibata T, Sako M, Maenaka K, Koshiba T, Mizumura H, Oda T, Kawabata S. J Biol Chem. 2014 Sep 12; 289 (37): 25987-95. Specifically, 2C12 monoclonal antibody (mouse), which is an anti FC antibody, was used as a capture antibody, anti FC polyclonal antibody (rabbit) was used as a detection antibody, and horseradish peroxidase (HRP)-labeled anti rabbit IgG polyclonal antibody (goat) (Bio-Rad Laboratories, Inc.) was used as a secondary antibody. By using TMB One Component HRP Microwell Substrate (SURMODICS), absorbance at 450 nm (control at 630 nm) was measured. The rFC concentration in a sample was calculated by using a calibration curve which has been established in advance using purified FC. Furthermore, for a test in which the commercially available recombinant reagent A is used, the rFC concentration at the time of endotoxin measurement was lower than 162 ng/mL.

### C. Measurement of relative potency of endotoxin of Helicobacter pylori CA2

In a case in which the lysate reagent is used, the relative potency of the endotoxin of Helicobacter pylori CA2 was about 2,000 EU/µg (Table 1). On the basis of this result, it is found to be desirable that, when a recombinant reagent is used, the relative potency of endotoxin of Helicobacter pylori CA2 is 200 EU/µg or higher (Yutaka Kikuchi et al., Pharmaceutical and Medical Device Regulatory Science, Vol. 48 (2017), No. 4, P252-260).

### (1) Endotoxin measuring reagent containing rFC produced with CHO DG44 cells (three factor system)

The relative potency of endotoxin of Helicobacter pylori CA2 was obtained from a three factor system containing rFC, rFB, and rPCE that were produced with CHO DG44 cells.

rFC, rFB, rPCE and a compound for detection (synthetic substrate Boc-LGR-pNA; PEPTIDE INSTITUTE, INC.) were added to Tris buffer solution to prepare an endotoxin measuring reagent. The rFC concentration in each endotoxin measuring reagent was adjusted by varying an addition amount of the enzyme solution of rFC. The measurement reagent was mixed, in a micro plate, with water for injection (blank) or a diluted solution of endotoxin to prepare a measurement sample. Furthermore, the preparation was made such that the concentrations of RSE and endotoxin of Helicobacter pylori CA2 in a measurement sample were 0.025 EU/mL and 0.25 ng/mL, respectively. Measurement of the relative potency was carried out on the basis of Example 1 (commercially available recombinant reagent A). Furthermore, rFB and rPCE were prepared so as to have the same concentration in each measurement sample.

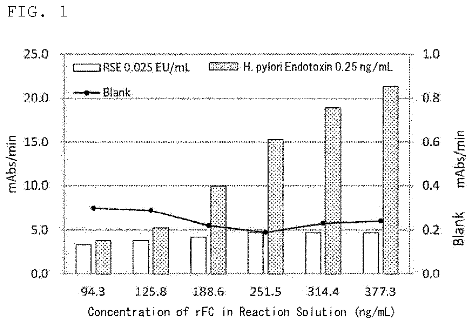

From 2 points between the blank and RSE, a calibration curve was established, and from the absorbance change ratio of endotoxin of Helicobacter pylori CA2, endotoxin activity was measured. The relative potency (EU/µg) of endotoxin of Helicobacter pylori CA2 in the case of using each endotoxin measuring reagent was obtained by dividing the endotoxin activity value (EU/mL), which has been obtained from above, by the concentration of the endotoxin of Helicobacter pylori CA2. As a result of plotting the rFC concentration and the relative potency and performing analysis, the relative potency of the endotoxin of Helicobacter pylori CA2 was 200 EU/µg or higher when the rFC concentration in the measurement sample was 162 ng/mL or more. Namely, it was found that the sensitivity was enhanced by increasing the rFC concentration in a measurement sample (Table 2 and FIG. 1). Furthermore, no increase in the absorbance change ratio was shown from the blank even when rFC concentration in the measurement sample was increased.

**Table 2**

| rFC produced with CHO DG44 cells (three factor system) | | | | |
|---|---|---|---|---|
| rFC | Absorbance change ratio (mAbs/min) | | | Relative potency (EU/µg) |
| ng/mL | Blank | RSE | H. pylori | |
| | | 0.025 EU/mL | 0.25 ng/mL | |
| 94.3 | 0.30 | 3.30 | 3.80 | 117 |
| 125.8 | 0.29 | 3.78 | 5.23 | 141 |
| 188.6 | 0.22 | 4.22 | 9.93 | 242 |
| 251.5 | 0.19 | 4.73 | 15.33 | 333 |
| 314.4 | 0.23 | 4.74 | 18.88 | 414 |
| 377.3 | 0.24 | 4.69 | 21.32 | 473 |

### (2) Endotoxin measuring reagent containing rFC produced with CHO DG44 cells (two factor system)

The relative potency of endotoxin of Helicobacter pylori CA2 was obtained from a two factor system containing rFC and rFB that were produced with CHO DG44 cells (two factor system not containing rPCE).

rFC, rFB, and a compound for detection (synthetic substrate Boc-LTR-MCA (namely, Boc peptidized AMC); PEPTIDE INSTITUTE, INC.) were added to Tris buffer solution to prepare an endotoxin measuring reagent. The rFC concentration in each endotoxin measuring reagent was adjusted by varying an addition amount of the enzyme solution of rFC. The measurement reagent was mixed, in a micro plate, with water for injection (blank) or a diluted solution of endotoxin to prepare a measurement sample. Furthermore, the preparation was made such that the concentrations of RSE and endotoxin of Helicobacter pylori CA2 in a measurement sample were 0.025 EU/mL and 0.25 ng/mL, respectively. Furthermore, rFB was prepared to have the same concentration in each the measurement sample. By using a fluorescence microplate reader, the relative fluorescence intensity of a measurement sample (RFU₀) was measured (excitation wavelength of 380 nm, and fluorescence wavelength of 445 nm). After that, the measurement sample was incubated at 37°C for 90 minutes, and the relative fluorescence intensity (RFU₉₀) was measured again. RFU₀ (background) was subtracted from RFU₉₀ to calculate ΔRFU. From ΔRFU of RSE and the blank, ΔRFU of each blank was subtracted to obtain corrected ΔRFU_{RSE} (Blanked ΔRFU_{RSE}) and corrected ΔRFU_{blank} (Blanked ΔRFU_{blank}). From two points between corrected ΔRFU_{blank} (that is, 0) and corrected ΔRFU_{RSE} of the blank, a calibration curve was established. Next, from ΔRFU of the endotoxin of Helicobacter pylori CA2, ΔRFU of the blank was subtracted to obtain corrected ΔRFU_{Hpy}, and the endotoxin activity (EU/mL) was obtained from the calibration curve. The relative potency (EU/µg) of endotoxin of Helicobacter pylori CA2 was obtained on the basis of Example 2C.(1) for the case of using each endotoxin measuring reagent.

As a result, when rFC concentration was 235 ng/mL or more in a measurement sample, the relative potency of endotoxin of Helicobacter pylori CA2 was 200 EU/µg or higher. Namely, it was found that the sensitivity was enhanced by increasing the rFC concentration in a measurement sample (Table 3). Furthermore, ΔRFU of the blank was hardly increased even when rFC concentration in the measurement sample was increased.

**Table 3**

| rFC produced with CHO DG44 cells (two factor system) | | | |
|---|---|---|---|
| rFC | Blanked delta RFU | | Relative potency |
| ng/mL | RSE | H. pylori | |
| | 0.025 EU/mL | 0.25 ng/mL | (EU/µg) |
| 94.3 | 582 | 126 | 22 |
| 125.8 | 715 | 428 | 60 |
| 188.6 | 727 | 1090 | 150 |
| 251.5 | 802 | 1750 | 218 |
| 314.4 | 840 | 2497 | 297 |
| 377.3 | 837 | 2982 | 356 |

### (3) Endotoxin measuring reagent containing rFC produced with CHO DG44 cells (one factor system)

The relative potency of endotoxin of Helicobacter pylori CA2 was obtained from a one factor system containing rFC that is produced with CHO DG44 cells (one factor system not containing rFB or rPCE).

rFC and a compound for detection (synthetic substrate Boc-VPR-MCA (namely, Boc peptidized AMC); PEPTIDE INSTITUTE, INC.) were added to Tris buffer solution to prepare an endotoxin measuring reagent. The rFC concentration in each endotoxin measuring reagent was adjusted by varying an addition amount of the enzyme solution of rFC. The measurement reagent was mixed, in a micro plate, with water for injection (blank) or a diluted solution of endotoxin to prepare a measurement sample. Furthermore, the preparation was made such that the concentrations of RSE and endotoxin of Helicobacter pylori CA2 in a measurement sample were 5 EU/mL and 25 ng/mL, respectively. The relative potency (EU/µg) of endotoxin of Helicobacter pylori CA2 was obtained on the basis of Example 2C.(2) for the case of using each endotoxin measuring reagent.

As a result, the relative potency of the endotoxin of Helicobacter pylori CA2 was 200 EU/µg or higher when rFC concentration in the measurement sample was 13 ng/mL or more. Namely, it was found that the sensitivity was enhanced by increasing the rFC concentration in a measurement sample (Table 4). Furthermore, ΔRFU of the blank was hardly increased even when rFC concentration in the measurement sample was increased.

**Table 4**

| rFC produced with CHO DG44 cells (one factor system) | | | |
|---|---|---|---|
| rFC | Blanked delta RFU | | Relative potency (EU/µg) |
| ng/mL | RSE | H. pylori | |
| | 5 EU/mL | 25 ng/mL | |
| 6.3 | 453 | 122 | 54 |
| 9.4 | 656 | 399 | 122 |
| 12.6 | 832 | 754 | 181 |
| 15.7 | 1223 | 1606 | 263 |

### (4) Endotoxin measuring reagent containing rFC produced with Expi CHO Expression System (three factor system)

The relative potency of endotoxin of Helicobacter pylori CA2 was obtained from a three factor system containing rFC, rFB, and rPCE that were produced with Expi CHO Expression System (transient expression system).

The relative potency of endotoxin of Helicobacter pylori CA2 was obtained on the basis of Example 2C.(1). As a result, the relative potency was 200 EU/µg or higher when rFC concentration in the measurement sample was 743 ng/mL or more. Namely, it was found that the sensitivity was enhanced by increasing the rFC concentration in a measurement sample (Table 5). Furthermore, no increase in the absorbance change ratio was shown from the blank even when rFC concentration in the measurement sample was increased.

**Table 5**

| rFC produced with Expi CHO Expression System (three factor system) | | | | |
|---|---|---|---|---|
| rFC | Absorbance change ratio (mAbs/min) | | | Relative potency (EU/µg) |
| ng/mL | Blank | RSE | H. pylori | |
| | | 0.025 EU/mL | 0.25 ng/mL | |
| 669.1 | 0.27 | 4.85 | 8.45 | 178 |
| 836.4 | 0.25 | 4.97 | 11.03 | 228 |
| 1003.7 | 0.24 | 4.84 | 12.23 | 261 |
| 1170.9 | 0.27 | 4.95 | 13.04 | 273 |

### (5) Endotoxin measuring reagent containing rFC produced with Expi CHO Expression System (one factor system)

The relative potency of endotoxin of Helicobacter pylori CA2 was obtained from a one factor system containing rFC that is produced with Expi CHO Expression System (transient expression system) (one factor system not containing rFB or rPCE).

The relative potency of endotoxin of Helicobacter pylori CA2 was obtained on the basis of Example 2C.(3). As a result, the relative potency was 200 EU/µg or higher when rFC concentration in the measurement sample was 77 ng/mL or more. Namely, it was found that the sensitivity was enhanced by increasing the rFC concentration in a measurement sample (Table 6). Furthermore, ΔRFU of the blank was hardly increased even when rFC concentration in the measurement sample was increased.

**Table 6**

| rFC produced with Expi CHO Expression system (one factor system) | | | |
|---|---|---|---|
| rFC | Blanked delta RFU | | Relative potency (EU/µg) |
| ng/mL | RSE | H. pylori | |
| | 5 EU/mL | 25 ng/mL | |
| 16.7 | 653 | 39 | 12 |
| 20.9 | 1053 | 104 | 20 |
| 41.8 | 2374 | 731 | 62 |
| 83.6 | 4355 | 4925 | 226 |

### (6) Endotoxin measuring reagent containing rFC produced with Sf9 cells (three factor system)

The relative potency of endotoxin of Helicobacter pylori CA2 was obtained from a three factor system containing rFC, rFB, and rPCE that were produced with Sf9 cells.

The relative potency of endotoxin of Helicobacter pylori CA2 was obtained on the basis of Example 2C.(1). As a result, the relative potency was 200 EU/µg or higher when rFC concentration in the measurement sample was 394 ng/mL or more. Namely, it was found that the sensitivity was enhanced by increasing the rFC concentration in a measurement sample (Table 7). Furthermore, no increase in the absorbance change ratio was shown from the blank even when rFC concentration in the measurement sample was increased.

**Table 7**

| rFC produced with Sf9 (three factor system) | | | | |
|---|---|---|---|---|
| rFC | Absorbance change ratio (mAbs/min) | | | Relative potency (EU/µg) |
| ng/mL | Blank | RSE | H. pylori | |
| | | 0.025 EU/mL | 0.25 ng/mL | |
| 185.5 | 0.38 | 5.56 | 3.96 | 69 |
| 247.4 | 0.35 | 5.25 | 5.35 | 102 |
| 309.2 | 0.35 | 4.78 | 6.37 | 136 |
| 371.0 | 0.36 | 3.86 | 6.04 | 162 |
| 495.1 | 0.38 | 2.75 | 8.99 | 363 |
| 618.9 | 0.38 | 1.84 | 9.16 | 601 |

### (7) Endotoxin measuring reagent containing rFC produced with Sf9 cells (one factor system)

The relative potency of endotoxin of Helicobacter pylori CA2 was obtained from a one factor system containing rFC that is produced with Sf9 cells (one factor system neither containing rFB nor rPCE).

The relative potency of endotoxin of Helicobacter pylori CA2 was obtained on the basis of Example 2C.(3). Here, the preparation was made such that the concentrations of RSE and endotoxin of Helicobacter pylori CA2 in a measurement sample were 0.25 EU/mL and 0.25 ng/mL, respectively. As a result, the relative potency was 200 EU/µg or higher when rFC concentration in the measurement sample was 93 ng/mL or more. Namely, it was found that the sensitivity was enhanced by increasing the rFC concentration in a measurement sample (Table 8). Furthermore, ΔRFU of the blank was hardly increased even when rFC concentration in the measurement sample was increased.

**Table 8**

| rFC produced with Sf9 (one factor system) | | | |
|---|---|---|---|
| rFC | Blanked delta RFC | | Relative potency (EU/µg) |
| ng/mL | RSE | H. pylori | |
| | 0.25 EU/mL | 0.25 ng/mL | |
| 65.0 | 469 | 83 | 177 |
| 86.7 | 527 | 99 | 188 |
| 130.0 | 640 | 177 | 277 |
| 173.3 | 789 | 287 | 364 |
| 216.7 | 873 | 450 | 515 |
| 260.0 | 904 | 602 | 666 |

### <Example 3: Influence of rFC content on sensitivity to endotoxin of Helicobacter pylori CA2>

### A. Preparation of recombinant protein

On the basis of Example 2A.(1), rFC of Limulus Polyphemus, L.p. was expressed using Chinese hamster ovarian cell line (CHO DG44 cells) as a host to obtain an enzyme solution of L.p. rFC. Furthermore, SEQ ID NO: 11 was used as DNA encoding FC of L.p.

### B. Measurement of L.p. rFC concentration

The L.p. rFC concentration was measured on the basis of Example 2B. The L.p. rFC concentration in a sample was calculated by using a calibration curve which has been established in advance using purified L.p. rFC.

### C. Measurement of relative potency of endotoxin of Helicobacter pylori CA2

### (1) Endotoxin measuring reagent containing L.p. rFC produced with CHO DG44 cells (one factor system)

The relative potency of endotoxin of Helicobacter pylori CA2 was obtained from a one factor system containing L.p. rFC that was produced with CHO DG44 cells (one factor system not containing rFB or rPCE). Furthermore, the preparation was made such that the concentrations of RSE and endotoxin of Helicobacter pylori CA2 in a measurement sample were 5 EU/mL and 25 ng/mL, respectively.

The relative potency (EU/µg) of endotoxin of Helicobacter pylori CA2 was obtained on the basis of Example 2C.(3). When the L.p. rFC concentration in a reaction solution was gradually increased from 45.8 ng/mL to 457.9 ng/mL, the relative potency (EU/µg) of endotoxin of Helicobacter pylori CA2 was increased from 33 EU/µg to 917 EU/µg. As a result of plotting the rFC concentration and the relative potency and performing analysis, the relative potency of the endotoxin of Helicobacter pylori CA2 was 200 EU/µg or higher when the L.p. rFC concentration in the measurement sample was 133 ng/mL or more. Namely, also in measurement of one factor system using L.p. rFC, it was found that the sensitivity is enhanced by increasing the L.p. rFC concentration in a measurement sample.

### <Example 4: Influence of rFC content on sensitivity to endotoxin of Helicobacter pylori CA2>

As rFC, rFC Enzyme Solution contained in the commercially available recombinant reagent B (PyroGene, LONZA JAPAN) was used.

### A. Measurement of rFC concentration in rFC Enzyme Solution

The rFC concentration was measured on the basis of Example 2B. The rFC concentration in a sample was calculated by using a calibration curve which had been established in advance using purified Carcinoscorpius rotundicauda, C.r.) rFC.

### B. Measurement of relative potency of endotoxin of Helicobacter pylori CA2

### (1) Endotoxin measuring reagent containing rFC (one factor system)

The relative potency of endotoxin of Helicobacter pylori CA2 was obtained from a one factor system using rFC Enzyme Solution (one factor system not containing rFB or rPCE). Furthermore, the preparation was made such that the concentrations of RSE and endotoxin of Helicobacter pylori CA2 in a measurement sample were 0.025 EU/mL and 5 ng/mL, respectively.

The relative potency (EU/µg) of endotoxin of Helicobacter pylori CA2 was obtained on the basis of Example 2C.(3). When the rFC concentration in a reaction solution was gradually increased from 203.7 ng/mL to 1,426 ng/mL, the relative potency (EU/µg) of endotoxin of Helicobacter pylori CA2 was increased from 9.09 EU/µg to 440 EU/µg. As a result of plotting the rFC concentration and the relative potency and performing analysis, the relative potency of the endotoxin of Helicobacter pylori CA2 was 200 EU/µg or higher when the rFC concentration in the measurement sample was 858 ng/mL or more. Namely, it was found that the sensitivity was enhanced by increasing the rFC concentration in a measurement sample even in the case of using a commercially available recombinant reagent.

### <Reference Example 1: Influence of rFB content on sensitivity to endotoxin of Helicobacter pylori CA2>

A measurement sample having a different rFB content was prepared, and the relative potency of endotoxin of Helicobacter pylori CA2 was obtained on the basis of Example 2C.(1). Furthermore, the preparation was made such that rFC and rPCE had the same concentrations in each measurement sample.

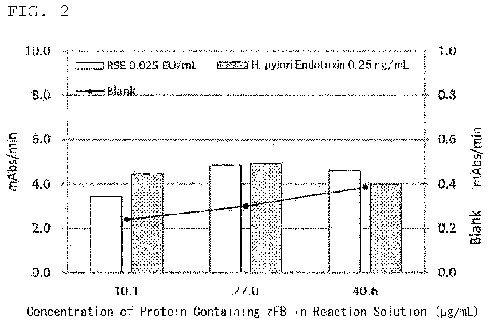

As a result, when the rFB concentration in a measurement sample is increased, the absorbance change ratio was increased in the blank and RSE, but the absorbance change ratio of endotoxin of Helicobacter pylori CA2 was hardly changed. Namely, it was found that the sensitivity to endotoxin of Helicobacter pylori CA2 was not enhanced even when the rFB concentration in a measurement sample was increased (FIG. 2).

### <Reference Example 2: Influence of rPCE content on sensitivity to endotoxin of Helicobacter pylori CA2>

A measurement sample having a different rPCE content was prepared, and relative potency of endotoxin of Helicobacter pylori CA2 was obtained on the basis of Example 2C.(1). Furthermore, the preparation was made such that rFC and rFB had the same concentrations in each measurement sample.

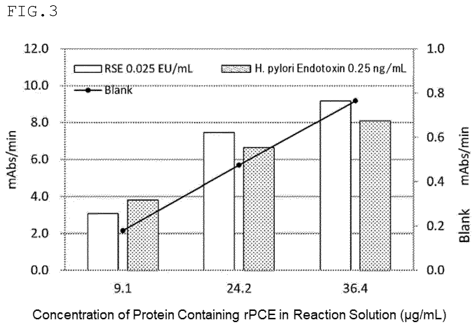

As a result, when the rPCE concentration in the measurement sample was increased, the absorbance change ratio was simultaneously increased in the blank, RSE, and endotoxin of Helicobacter pylori CA2. Namely, it was found that the sensitivity to endotoxin of Helicobacter pylori CA2 was not enhanced even when the rPCE concentration in a measurement sample was increased (FIG. 3).

### Industrial Applicability

According to the present invention, a method for enhancing the sensitivity of an endotoxin measuring reagent employing a recombinant protein to the endotoxin of Helicobacter pylori can be provided. Accordingly, the present invention is to provide an endotoxin measuring method having reduced influence caused by a difference in origin of endotoxin in which an endotoxin measuring reagent employing a recombinant protein is used.

### <Description of Sequence Listing>

SEQ ID NO: 1: cDNA base sequence of factor C of Tachypleus tridentatus
SEQ ID NO: 2: Amino acid sequence of factor C of Tachypleus tridentatus
SEQ ID NO: 3: cDNA base sequence of factor C of Carcinoscorpius rotundicauda
SEQ ID NO: 4: Amino acid sequence of factor C of Carcinoscorpius rotundicauda
SEQ ID NO: 5: cDNA base sequence of factor B of Tachypleus tridentatus
SEQ ID NO: 6: Amino acid sequence of factor B of Tachypleus tridentatus
SEQ ID NO: 7: cDNA base sequence of pro-clotting enzyme of Tachypleus tridentatus
SEQ ID NO: 8: Amino acid sequence of pro-clotting enzyme of Tachypleus tridentatus
SEQ ID NO: 9: cDNA base sequence of factor B of Tachypleus tridentatus in which codons are optimized for expression in insect cells
SEQ ID NO: 10: cDNA base sequence of factor C of Limulus polyphemus
SEQ ID NO: 11: cDNA base sequence of factor C of Limulus polyphemus in which codons are optimized for expression in CHO cells
SEQ ID NO: 12: Amino acid sequence of factor C of Limulus polyphemus

This application claims priority from Japanese Patent Application No. 2018-188587 filed October 3, 2018, the entire contents of which are hereby incorporated by reference herein.

The present invention relates to the following items:
1. A method for enhancing a sensitivity of an endotoxin measuring reagent to the endotoxin of Helicobacter pylori,
   the endotoxin measuring reagent comprising a recombinant protein of horseshoe crab factor C,
   the method comprising increasing a content of the recombinant protein of factor C at the time of endotoxin measurement.
2. The method according to item 1, wherein the endotoxin measuring reagent comprises a recombinant protein of horseshoe crab factor B.
3. The method according to item 1 or 2, wherein the endotoxin measuring reagent comprises a recombinant protein of horseshoe crab pro-clotting enzyme.
4. The method according to any one of items 1 to 3, wherein the endotoxin measuring reagent comprises a compound for detection.
5. An endotoxin measuring method in which sensitivity of an endotoxin measuring reagent to the endotoxin of Helicobacter pylori is enhanced, the method comprising:
   preparing an endotoxin measurement sample comprising the endotoxin measuring reagent and an analyte,
   wherein the endotoxin measuring reagent comprises a recombinant protein of horseshoe crab factor C, and
   the sensitivity is enhanced by increasing a concentration of the recombinant protein of factor C in the endotoxin measurement sample.
6. The method according to item 5, wherein the endotoxin measuring reagent comprises a recombinant protein of horseshoe crab factor B.
7. The method according to item 5 or 6, wherein the endotoxin measuring reagent comprises a recombinant protein of horseshoe crab pro-clotting enzyme.
8. The method according to any one of items 5 to 7, wherein the endotoxin measuring reagent comprises a compound for detection.
9. A method for producing an endotoxin measuring reagent having enhanced sensitivity to the endotoxin of Helicobacter pylori,
   the endotoxin measuring reagent comprising a recombinant protein of horseshoe crab factor C,
   the method comprising increasing a content of the recombinant protein of factor C in the endotoxin measuring reagent.
10. An endotoxin measuring reagent comprising a horseshoe crab factor C,
   wherein the horseshoe crab factor C is a recombinant protein, and
   relative potency of endotoxin of Helicobacter pylori is 200 (EU/µg) or more.
11. The endotoxin measuring reagent according to item 10, comprising a recombinant protein of horseshoe crab factor B, a recombinant protein of horseshoe crab pro-clotting enzyme, and a compound for detection represented by general formula Y-X-Z,
   wherein in the above general formula, Y is a hydrogen atom or a protecting group, X is a peptide comprising the amino acid sequence, which is a substrate of the recombinant protein of pro-clotting enzyme, and Z is a labeling material which becomes optically detectable when being released from X.
12. The endotoxin measuring reagent according to item 10, comprising a recombinant protein of horseshoe crab factor B and a compound for detection represented by general formula Y-X-Z,
   wherein in the above general formula, Y is a hydrogen atom or a protecting group, X is a peptide comprising the amino acid sequence, which is a substrate of the recombinant protein of factor B, and Z is a labeling material which becomes optically detectable when being released from X.
13. The endotoxin measuring reagent according to item 10, comprising a compound for detection represented by general formula Y-X-Z,
   wherein in the above general formula, Y is a hydrogen atom or a protecting group, X is a peptide comprising the amino acid sequence, which is a substrate of the factor C, and Z is a labeling material which becomes optically detectable when being released from X.

## Claims

1. A method for enhancing a sensitivity of an endotoxin measuring reagent to the endotoxin of *Helicobacter pylori,*
the endotoxin measuring reagent comprising a recombinant protein of horseshoe crab factor C, a recombinant protein of horseshoe crab factor B, and a recombinant protein of horseshoe crab pro-clotting enzyme,
the method comprising increasing a content of the recombinant protein of factor C to 162 ng/mL or more at the time of endotoxin measurement.

2. The method according to claim 1, wherein the endotoxin measuring reagent comprises a compound for detection.

3. The method according to claim 2, wherein the compound for detection is represented by the general formula Y-X-Z,
wherein in the above general formula, Y is a hydrogen atom or a protecting group, X is a peptide containing an amino acid sequence which is a substrate of the recombinant protein of pro-clotting enzyme, and Z becomes a labeling material which is optically detectable when being released from X.

4. The method according to any one of claims 1 to 3, wherein the horseshoe crab is *Tachypleus tridentatus, Carcinoscorpius rotundicauda,* or *Limulus polyphemus.*
